# EUROPEAN PATENT APPLICATION

(11) **EP 4 595 953 A1**
(43) Date of publication of application: **06.08.2025**
(21) Application number: 24154574.8
(22) Date of filing: 30.01.2024
(51) Int. Cl.: A61K 9/10, A61K 31/58, A61K 47/38, A61P 1/04

(54) **ORALLY APPLICABLE SUSPENSION FOR THE TREATMENT OF EOSINOPHILIC ESOPHAGITIS IN CHILDREN**

(71) Applicant: Dr. Falk Pharma GmbH, 79108 Freiburg (DE)
(72) Inventor: Müller, Ralph, 79111 Freiburg (DE); Burrack, Sarah, 79100 Freiburg im Breisgau (DE); Greinwald, Roland, 79341 Kenzingen (DE); Wilhelm, Rudolf, 76476 Bischweier (DE); Korostylev, Alexander, 79114 Freiburg im Breisgau (DE)
(74) Representative: Maucher Jenkins Patent- und Rechtsanwälte

(57) **Abstract**

Disclosed is an orally applicable suspension comprising
a) topically acting micronized budesonide,
b) an aqueous base,
c) at least one viscosity-increasing agent selected from a methylcellulose or a derivative thereof,
d) a buffering agent which allows the adjustment of the final suspension to a pH value of 3.5 to 4.5,
e) an antimicrobial preservative,
f) a suspension stabilizing agent, in particular ethylenediaminetetramethyl acetic acid,
whereby the suspension has a dynamic viscosity in the range of 800 mPa*s to 2000 mPa*s and a process for preparing such solution.

## Description

Eosinophilic esophagitis (EoE) is a chronic immune/antigen-mediated disorder of the esophagus characterized histologically by eosinophil-predominant mucosal inflammation and clinically by symptoms of esophageal dysfunction. Its incidence in children has been increasing and is currently similar to that of pediatric inflammatory bowel disease (IBD). EoE involves progressive esophageal remodeling that can lead to esophageal dysmotility and strictures, if untreated. Therapeutic targets currently include improvement of symptoms, remission of histopathological features and prevention of long-term complications (Oliva et al., Digestive Diseases and Sciences (2019), 64; 1571-1578).

Topical corticosteroids (tCS), predominantly fluticasone and budesonide, are considered the most effective first-line treatment as well as an option for maintenance therapy in EoE. These drugs induce histological remission, lead to the resolution of clinical symptoms and prevent fibrosis. The way that topical corticosteroids are administered significantly affects their effectiveness. There is no commercially available steroid drug formulation to be applied topically to the esophagus in children. For adults with EoE, budesonide orodispersible tablets (i.e. Jorveza^{®}) were recently designed. In children, however, swallowed metered-dose inhalers (MDI) steroids were used.

EP 3 834 819 describes the treatment of a six-year old male patient with oral prednisone and the treatment of a five-year old girl with Pulmicort^{®} mixed with sucralose.

Clinical studies were limited to swallowing aerosolized or nebulized corticosteroid formulations that were developed for asthma. Dellon ES, Sheik O, et al. disclose that viscous topical therapy is more effective than nebulized steroid therapy for patients with eosinophilic esophagitis (Gastroenterology 2012; 143:321-324). For topical corticosteroid therapy of EoE in children and adolescents, fluticasone or budesonide was used. Budesonide was prepared as aqueous solution mixed with sucralose resulting in a viscous solution. Sometimes, instead of sucralose, Duocal^{®} was used which is a viscous mixture of oil and glucose (Claßen, Gastro-News 2017, 04(1), page 26).

For the treatment of children, in particular young children, it is difficult to find a pharmaceutical solution which is accepted by children and their caregivers and which provides the desired healing effects.

EoE is a chronic, immune/antigen-mediated disease characterized clinically by symptoms related to esophageal dysfunction and histologically by eosinophil-predominant inflammation exclusively in the esophageal mucosa. The disease affects not only adults but also young children with 1 year up to 18 years and adolescents. The major challenge of the treatment of EoE is the identification and selection of a suitable dosage form that considers the physico-chemical characteristics of the active pharmaceutical ingredient and that facilitates its delivery to the affected areas of the esophagus after oral administration to ensure topical treatment, i.e. the drug product should be orally applied but locally acting in the esophagus. While these attributes of the dosage form are age-independent and apply to the entire patient population, there are special and critical features that are unique to the pediatric patient population. The general and critical features or quality attributes require the selection of a suitable age-appropriate pediatric dosage form providing the following characteristics:
- The age-appropriate pediatric dosage form must be accepted by the target population and their caregivers, in order to ensure compliance with the treatment regimen ("acceptability").
- The age-appropriate pediatric dosage form must allow a flexible and safe dosing regimen that takes into account the developmental status of the various pediatric populations ("dosing flexibility").
- The age-appropriate pediatric dosage form must deliver the active pharmaceutical ingredient to the target site of action, i.e. esophagus ("esophagus targeting") where it should be available to exert its efficacy ("biopharmaceutical fitness").
- The age-appropriate pediatric dosage form must ensure a sustainable/lasting availability of the active pharmaceutical ingredient at the target site of action after swallowing the formulation considering that fractions of the dosage form which have moved into the stomach are lost to contribute to the efficacy of the active pharmaceutical ingredient ("sustainability").
- The age-appropriate pediatric dosage form must be physically, chemically and microbiologically stable and manufacturable in order to be commercially viable ("commercial viability").

Budesonide is a potent glucocorticosteroid with a high topical anti-inflammatory activity and with low systemic effects. It has been widely used as active pharmaceutical ingredient for the treatment of asthma or inflammatory bowel diseases such as Crohn's disease and ulcerative colitis. In addition, it is also used to treat EoE in adults.

Different budesonide formulations and dosage forms that target the lung or different regions of the gastrointestinal tract have been developed over the last decades. They are already commercially available and marketed worldwide. However, none of these drug products can be considered as a suitable age-appropriate pediatric dosage form that meets the above-mentioned critical quality attributes in its entirety, and none of these drug products is suitable to treat eosinophilic esophagitis in children.

In addition, liquid and solid formulations are generally considered as the first choice when selecting an age-appropriate pediatric dosage form for oral administration. However, budesonide tends to be susceptible to chemical degradation in liquid and semi-solid formulations especially when not sufficiently stabilized. This strongly limits the number of potential age-appropriate pediatric dosage forms of budesonide. Therefore, and opposed to adults, children are currently excluded from a suitable treatment of eosinophilic esophagitis with a budesonide-containing drug product meeting all the required features.

The major challenge of the development was the identification and selection of a suitable age-appropriate pediatric dosage form considering the physico-chemical properties of budesonide and meeting the core enabling criteria for appropriateness such as acceptability, dosing flexibility, esophagus targeting, biopharmaceutical fitness, sustainability and commercial viability.

From a stability point of view solid dosage forms are generally preferred over liquid dosage forms. Potential formulations are powders and granules, tablets or minitablets, orodispersible tablets, chewable or effervescent tablets and capsules. When administered to children, however, all these formulations will quickly pass the esophagus after swallowing and deliver budesonide predominantly into the stomach. Esophagus targeting, the essential criterion for the selection of a suitable dosage form to treat eosinophilic esophagitis will not be achieved in the target pediatric population.

It is an object of the present invention to provide a stable viscous suspension of budesonide which allows good treatment results, in particular high rates of remission which should be achieved with the use of a low concentration of budesonide. An important aspect thereby is that the viscous budesonide suspension provides improved adherence of the suspension containing the budesonide at the inflamed parts of the esophagus which are frequently located in the area where the esophagus enters the stomach.

Surprisingly, a stable liquid dosage form of budesonide could be developed that overcame the described obstacles of the solid dosage forms. The especially preferred age-appropriate pediatric dosage form to target the esophagus is a highly viscous aqueous suspension of budesonide in a concentration of about 0.2 mg/mL that is presented in a 200 mL amber glass bottle (type III glass) as multi-dose container with a child-resistant closure (white LDPE screw cap with an internal colorless LDPE flow limiter). The required doses are withdrawn from the bottle using a suitable measuring device (i.e. oral syringe) that also allows flexible dosing. After first opening, the flow limiter remains in the bottle neck and enables the insertion of the oral syringe for withdrawal of the suspension. The drug product is stable under long-term storage conditions and provides a sufficiently long in-use stability after first opening.

Usually, an oral suspension comprises the suspended active pharmaceutical ingredient with defined characteristics in terms of crystallinity and particle size distribution, the viscosity-increasing agent that affects the physical stability of the formulation and the components of the outer phase of the suspension affecting the chemical stability, preservation, organoleptic properties and safety of the formulation. The invention comprises an aqueous oral suspension of the active pharmaceutical ingredient budesonide which is per se practically insoluble in water. As the vehicle is already saturated with dissolved budesonide, the undissolved drug particles are immediately available for dissolution after delivery to the esophagus.

The present invention in its broadest sense relates to an orally applicable suspension comprising the following components:
A topically acting micronized crystalline glucocorticosteroide for the present invention is budesonide.

The orally applicable suspension is aqueous. The main component is therefore purified water which can be used for pharmaceutical purposes. Another very important component is a viscosity-increasing agent which is in the present case selected from methylcellulose or a derivative thereof. The methylcellulose preferably used in the present invention is a methyl ester of cellulose that contains about 20-50% of methoxy groups. Methylcellulose is soluble in water and a wide range of viscosity grades can be obtained depending on the average molecular weight range which is between 10000 and 220000 Da. Depending on the particular type of methylcellulose the amount required for obtaining a dynamic viscosity in the range of 800 mPa*s to 2000 mPa*s is added. The amount of the methylcellulose which is added to the solution is preferably less than 2% weight/weight. The viscosity is influenced by the length of the cellulose chain and the degree of methylation.

Methylcellulose is a nonionic water-soluble cellulose ether where some of the hydroxy groups of cellulose are substituted with methyl groups leading to methoxy groups. It is a pharmacopoeial excipient and described in the European Pharmacopoeia (Ph. Eur.), the Pharmacopoeia of the United States (USP) and other Pharmacopoeias. As partly O-methylated cellulose the content of methyl groups ranges from 26.0 per cent to 33.0 per cent. The average degree of substitution is 1.5 ranging from 1.4 to 2.0 so that on average one glucopyranose unit contains 1.5 methoxy groups. Methylcellulose is a polymer comprising numerous linked glucopyranose units with a mean chain length of 200 to 1000 and relative molecular weights of 20,000 to 150,000. The viscosity of methylcellulose solutions is related to the concentration and molecular weight of the polymer.

Different grades and viscosity specifications of methylcellulose are available. The manufacturers of the polymer usually classify the grades by the viscosity of 2% aqueous solutions measured at 20°C. Available grades and viscosity specifications are presented below:

| | | |
|---|---|---|
| • Methylcellulose, 4 mPa*s | 3.2 - 4.8 mPa*s | (range: 80 - 120%) |
| • Methylcellulose, 15 mPa*s | 12.0 - 18.0 mPa*s | (range: 80 - 120%) |
| • Methylcellulose, 25 mPa*s | 22.0 - 30.0 mPa*s | (range: 80 - 120%) |
| • Methylcellulose, 100 mPa*s | 80 - 120 mPa*s | (range: 80 - 120%) |
| • Methylcellulose, 400 mPa*s | 320 - 480 mPa*s | (range: 80 - 120%) |
| • Methylcellulose, 1500 mPa*s | 1125 - 2100 mPa*s | (range: 75 - 140%) |
| • Methylcellulose, 4000 mPa*s | 3000 - 5600 mPa*s | (range: 75 - 140%) |

Methylcellulose dissolves in cold water giving a colloidal solution. Despite its insolubility in hot water, the hot water method is preferably used to prepare the colloidal solutions. After dispersing and wetting the polymer in hot water, the mixture is cooled down so that complete dissolution can be achieved that results in a clear solution. The invented budesonide 0.2 mg/mL oral suspension is preferably manufactured using methylcellulose grade 1500 mPa*s at a concentration of 20 mg/mL. However, adjustment of the visocsity can also be accomplished with other methylcellulose grades or with combinations or blends of different methylcellulose grades. Thus the preferred target viscosity of 1000 mPa*s (within the range of 800 to 1200 mP*s) of the drug product can be reliably adjusted.

Those methylcelluloses are usually used in the oral application suspension in a range from 10 mg/ml to 50 mg/ml, preferably 15 mg/ml to 25 mg/ml and most preferred 20 mg/ml.

The orally applicable suspension must have a well-defined pH value whereby two aspects have to be observed. On the one hand the pH must be in a range where the suspended budesonide is highly stable and on the other hand the pH must be in a range which is well accepted when the suspension is applied to the patient. Many buffers used for pharmaceutical formulations are systems comprising carbonates, citrates, tartrates and various phosphate salts. In the present case a citrate buffering system is especially preferred.

The orally applicable suspension also contains preservatives which prevent the growth of undesired microorganisms like bacteria or fungi in the suspension once it is opened for the first time. There is always a risk of microbial contamination by applying the syrup with a syringe. Therefore, a preservative is used in the present case which is well tolerable for the target patient group. Suitable antimicrobial preservatives for oral liquid formulations are sorbic acid or its potassium salt, benzoic acid or its sodium salt, parahydroxybenzoate esters (especially methyl and ethyl) or their sodium salts and ethanol. They are used alone or in combinations. However, the selection of a suitable antimicrobial preservative or combination of preservatives should be carefully conducted when used in pediatric formulations. In particular preferred is sodium benzoate.

The dissolved and undissolved budesonide are stabilized by adjusting the pH value of the aqueous continuous external phase to 3.5 to 4.5 and by adding the disodium salt of ethylenediaminetetraacetic acid (EDTA) as stabilizer to the outer phase. Under these conditions the chemical and microbiological long-term stability of the suspension can be ensured.

It is of utmost importance that the orally applicable suspension has an appropriate viscosity. The viscosity is measured according to the methods as described in the European Pharmacopeia [2.2.10]. The viscosity ranges from 500 mPa*s to 2000 mPa*s, preferably from 800 mPa*s to 1200 mPa*s and particularly preferred to about 1000 mPa*s.

The concentration of the glucocorticosteroid is in the range of about 0.1-1.0 mg/ml budesonide. Particularly preferred ranges are from 0.1-0.5 mg/ml whereby 0.2 mg/ml is particularly preferred.

In order to allow a good suspension of the glucocorticosteroide, the pharmaceutically active agents are applied in a micronized form. This is a particular treatment of the budesonide whereby at least 95% of the budesonide microparticles have a diameter of less than 10 µm. Particularly preferred is that at least 95% of the budesonide microparticles have a diameter of less than 5 µm.

The selection of the appropriate dynamic viscosity is of high importance. Preferably the viscosity is in a range of 1000-15000 mPa*s and especially preferred is a viscosity of about 1200 mPa*s whereby the viscosity is dependent on the type of methylcellulose and not on the amount of viscosity enhancer.

The acceptance of the orally applicable suspension by the patient depends strongly on the taste. Whereas it is possible to use artificial sweeteners like sucralose in the present case a naturally occurring sweetener as sucrose is preferred.

Furthermore, the orally applicable suspension contains a flavoring agent whereby cassis aroma is particularly preferred.

Since the orally applicable suspension is to be applied to children it contains no surfactant and it contains no antioxidant. This can advantageously increase the willingness of patients or their caregivers to take medication with the orally applicable suspension according to the invention.

In order to avoid any undesired side effects, the orally applicable suspensions according to the invention contain no other viscosity-increasing agent except methylcellulose or derivatives thereof as described above. Different types of methylcellulose as described above can be mixed.

The orally applicable suspension according to the present invention is intended for use in the treatment of eosinophilic esophagitis in children who are patients having an age from about 1 to 2 years to about 17 years. The amount of the glucocorticosteroid, in particular the budesonide is 0.5 to 1 mg per dose which is administered in an amount of 2.5-5 ml suspension depending on the concentration of the orally applicable suspension. Such dose is applied preferably twice a day, namely one dose in the morning and one dose in the evening. By using this method of treatment acute phases of the disease can be treated and it is also possible to bring the patient into remission whereby the suspension is applied for at least 48 weeks.

The orally applicable suspension according to the invention is prepared in a special process whereby in a first process step an aqueous base is mixed with the viscosity-increasing agent producing a first suspension without the pharmaceutically active agent. The solution is usually produced by temperature treatment which means that the solution is slightly heated.

In a second step an aqueous solution containing a buffering agent, an antimicrobial preservative and a suspension stabilizing agent is prepared.

In a third step the solution containing the viscosity-increasing agent obtained from step 1 is mixed with the solution containing the buffering agent, an antimicrobial preservative and a suspension stabilizing agent and in a fourth step the micronized corticosteroid is suspended under stirring in the solution as described above. Finally, the flavoring agent is added before the budesonide solution is filled into light protected bottles. The preparation of the solution must be prepared by observing the strictest standards of good manufacturing practice for preparing syrups.

Technologically the invention comprises the following features:
- Micronized crystalline budesonide is suspended in an appropriate aqueous continuous external phase or vehicle having a pH value of 3.5 to 4.5. Within this pH range, budesonide is most stable and simultaneously optimal conditions for sodium benzoate are guaranteed. Moreover, the low solubility of the active pharmaceutical ingredient in water is beneficial so that long-term chemical and physical stability of the drug molecule can be ensured without the use of undesired stabilizing agents such as antioxidants. The properties of budesonide do not change upon storage and in-use so that the safety, efficacy and drug delivery remain unaltered.
- An inert and mucoadhesive nonionic cellulose derivative is used as suspending and thickening agent in order to increase and adjust a pH-independent but time-dependent shear-thinning/pseudoplastic viscosity of the vehicle. The suspending and viscosity-increasing agent is preferably methylcellulose, a partly O-methylated cellulose. The thixotropic properties of the vehicle facilitate (a) the applicability of the oral suspension in terms of withdrawal and pallatabiltiy, (b) the extension of the contact time of the oral suspension with the mucosa of the esophagus and (c) the homogeneous distribution of budesonide after moderate shaking (dose uniformity). The colloid also enables the dispersion of the solid particles in the liquid by forming physically stable suspensions upon shaking without the use of a wetting agent or surfactant. Omitting surfactant is beneficial for the patient since it reduces chances of irritation at the inflamed esophageal mucosa. Within the aqueous suspension, budesonide is only dissolved partially. The majority of active ingredient is undissolved and readily available at the target location. Together with the usage of micronized crystalline budesonide enabling drastically higher speed of dissolution, surfactants can be omitted.
   The dynamic viscosity of the oral suspension is preferably adjusted in the range of 800 mPa*s to 1200 mPa*s, preferably to 1000 mPa*s. The highly viscous oral suspension can be easily resuspended by avoiding foaming, air entrapment and rapid re-sedimentation. This avoids dosing errors due to an inhomogeneous distribution of the active pharmaceutical ingredient.
- The budesonide concentration of the oral suspension is preferably adjusted to 0.2 mg/mL resulting in an optimal dose volume ranging from 2.5 mL to 5 mL which equals the volume of a usual gulp. The dose volume, the bioadhesive nature of the colloid and the viscosity of the vehicle facilitate the lining of the pediatric esophagus with micronized crystalline budesonide after swallowing so that local activity of the active pharmaceutical ingredient can be increased thereby reducing loss of budesonide by passaging into the stomach. Uniform withdrawal of the dose is preferably achieved by using a 5 mL CE-certified oral syringe consisting of a barrel with an embossed graduation for 2.5 mL respectively 5 mL and a plunger.
- The components of the suspending vehicle comprise sucrose as sweetening agent, citric acid as buffering agent or acidity regulator, sodium benzoate as antimicrobial preservative, disodium edetate as stabilizing agent and Cassis aroma (Blackcurrant flavor) as flavoring agent. These components are compatible with the active pharmaceutical ingredient and the viscosity-increasing agent. The acceptance of the formulation by the pediatric population with regard to its organoleptic properties (taste and aroma) is greatly enhanced by the qualitative and quantitative composition of the oral suspension.
- The concentration of budesonide in the oral suspension is preferably 0.02%. The high viscosity of the vehicle, its pleasant taste and the use of micronized and low-concentrated budesonide avoids the feeling of grittiness in the mouth and thus greatly enhances the acceptance of the suspension to children.

The examples described in the following illustrate the present invention and preferred embodiments without, however, limiting the scope of protection. In the examples and in the present invention the following abbreviations are used:
- ADR - Adverse Drug Reaction
- AE - Adverse Events
- AESI - Adverse Event of Special Interest
- BID - Twice Daily
- BL - Baseline
- BOS - Budesonide Oral Suspension
- BOS-H - Budesonide Oral Suspension (High Dose)
- BOS-L - Budesonide Oral Suspension (Low Dose)
- BUU - Budesonide Suspension (= BOS)
- DB - Double-Blind
- EoEHSS - EoE histological scoring system
- eos - eosinophils
- EOT - End of Treatment
- REFS - Endoscopic Reference Score
- FAS - Full Analysis Set
- FU - Follow-Up
- hpf - high power field
- IMP - Investigational Medicinal Product
- LOCF - Last Observation Carried Forward
- OBS - Oral Budesonide Suspension
- OD - Once Daily
- OLE - Open Label Extension
- OLI - Open Label Induction
- PLAC - Placebo
- PP - per protocol
- SAE - Serious Adverse Event
- SCR - Screening
- TAP - Tapering

### Example 1

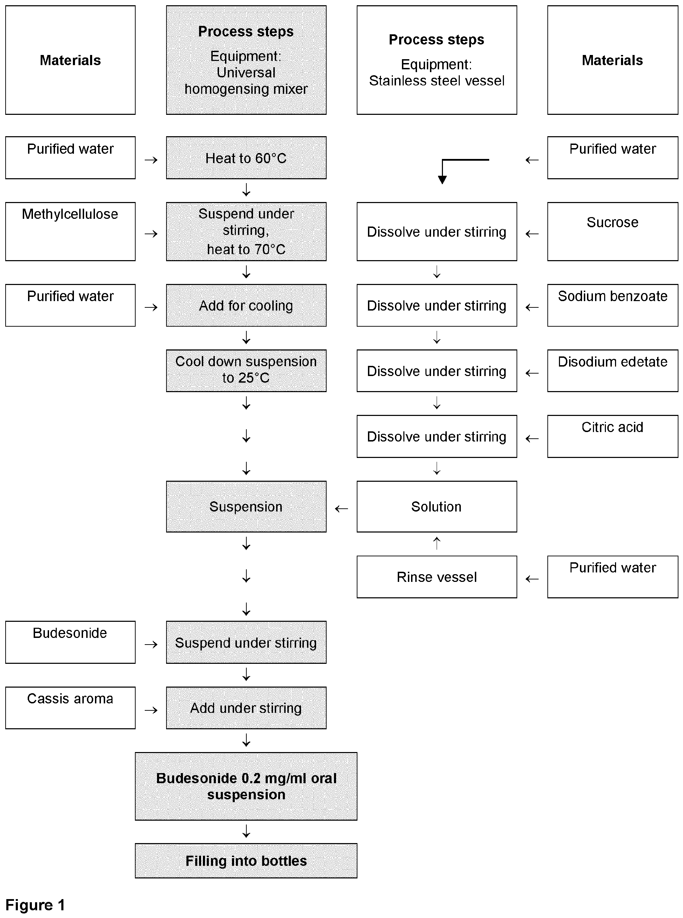

A scheme for the preparation of an orally applicable suspension according to the invention is shown in Fig. 1.

The preferred suspending and thickening agent of the invention is methylcellulose. Other pharmaceutically acceptable, inert and mucoadhesive nonionic cellulose derivatives could be used as suspending and thickening agent as minor component. Minor component means that such a component is present in an amount not exceeding 1 mg/ml, preferably not exceeding 0.5 mg/ml of the final orally applicable suspension. The advantage of methylcellulose is that the viscosity can be adjusted independently of the pH value. Thus, the viscosity can or is preferably adjusted at an acidic pH value, in particular in a range of pH 3.5 to 4.5. Moreover, it is of utmost importance that the solution when used by the patient sticks to the esophagus of the patient for sufficient time to develop its effect.

The orally applicable solution according to the invention sticks very well to patient's esophagus and it does not cause an uncomfortable feeling.

Budesonide 0.2 mg/mL oral suspension is a whitish, highly viscous suspension with a characteristic cassis flavor. One bottle of the 200 ml amber glass bottle contains > 160 ml of the liquid preparation. The drug product is usually manufactured in batches of approximately 434 kg resulting in 2500 bottles with a filling volume of 165 ml. The qualitative and quantitative composition of the invention per mL of the suspension, per dose of 5 mL and per batch of 434 kg are presented below.

Table 1 shows a preferred composition of the orally applicable solution according to the invention.

**Table 1**

| **Component** | **Qualitative and quantitative composition** | | |
|---|---|---|---|
| | [mg/mL] | [mg/5 ml] | [kg/batch] |
| Budesonide | 0.20 | 1.00 | 0.08250 |
| Sucrose | 100.00 | 500.00 | 41.2500 |
| Sodium benzoate | 0.86 | 4.30 | 0.35475 |
| Disodium edetate | 1.00 | 5.00 | 0.41250 |
| Citric acid, anhydrous | 0.70 | 3.50 | 0.28875 |
| Methylcellulose | 20.00 | 100.00 | 8.25000 |
| Blackcurrant flavour (Cassis aroma) | 0.80 | 4.00 | 0.33000 |
| Purified water | 928.84 | 4644.20 | 383.14650 |
| **Total:** | **1052.40** | **5262.00** | **434.11500** |

The manufacturing process of budesonide 0.2 mg/mL oral suspension comprises the process steps to prepare the bulk suspension and the filling process. The flow chart as shown in Fig. 1 describes the manufacturing process of the bulk suspension (batch size 434.115 kg).

### Example 2 (selection of the appropriate methylcellulose)

The described invention has been successfully tested in children in clinical studies. Several drug product batches were manufactured to supply the clinical trials. All the tested batches had the invented qualitative and quantitative composition as described above using methylcellulose, 1500 mPa*s with a concentration of 20 mg/ml (equivalent to 1.90%). The measured dynamic viscosity of the clinical batches is presented below (method: rotating viscosity according to Ph. Eur. 2.2.10, rotational speed = 35 sec⁻¹, 20°C, duration of measurement = 60 sec):

| | |
|---|---|
| • Batch V2188 | 1153 mPa*s |
| • Batch V2253 | 1015 mPa*s |
| • Batch V2270 | 1187 mPa*s |
| • Batch V3031 | 1186 mPa*s |
| • Batch V3086 | 1149 mPa*s |
| • Batch V3111 | 986 mPa*s |

The specific viscosity values were obtained by using appropriate amounts of methylcellulose having 1500 mPa*s.

### Example 3 (phase 2/3 clinical study)

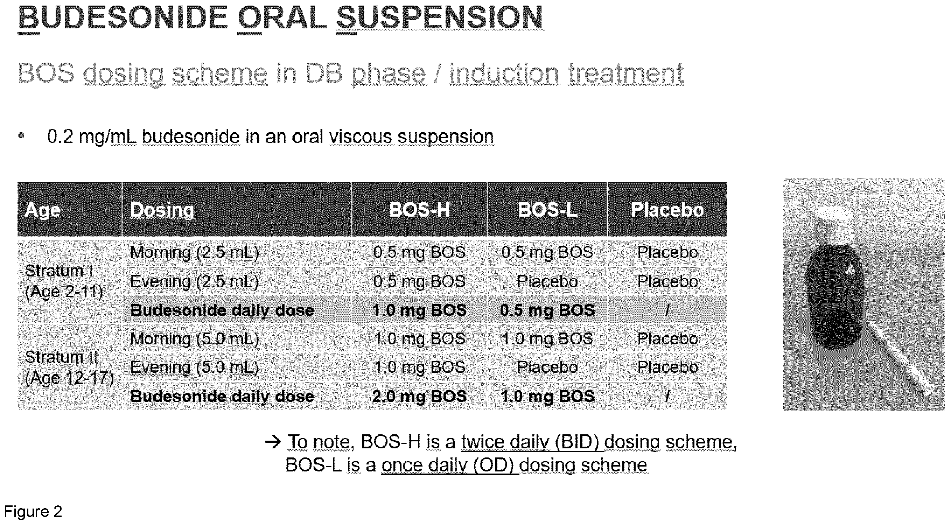

In the phase 2/3 clinical study BUU-5/EEA using the suspension described herein, two different dosing schemes were used. The dosing scheme is shown in Figure 2.

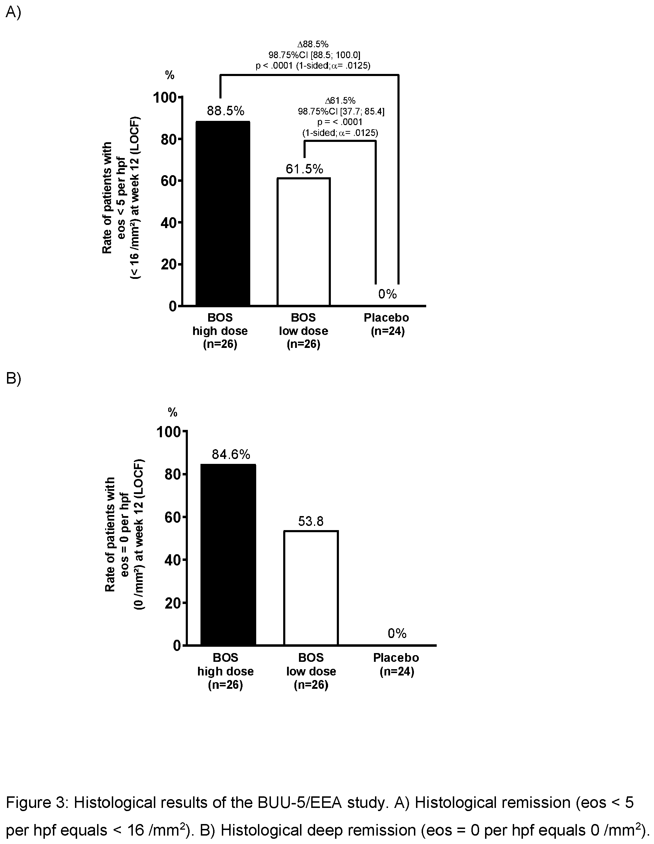

After 12 weeks of treatment the rate of patients with histological remission (i.e. eosinophil count of < 5 eosinophils per hpf / < 16 eosinophils per mm²) was determined. The results are shown in Figure 3A. It turned out that the high dose budesonide solution (0.5 - 1.0 mg, OD) resulted in very high percentage of histological remission after a treatment of 12 weeks only. The low dose (1.0 - 2.0 mg, BID) resulted also in a remission substantially higher than treatment with placebo but it seems that a high dosage budesonide treatment is better for obtaining a quick remission.

In addition, after 12 weeks of treatment the rate of patients with the more stringent eosinophil count of 0 eosinophils per hpf ("histological deep remission") was determined (Figure 3B).

A comparison between the two dosing schemes is shown in Table 2.

**Table 2**

| | according to the invention | |
|---|---|---|
| Treatment regimen | OD | BID |
| Daily dose | **0.5** - **1.0 mg** | **1.0** - **2.0 mg** |
| Volume | 1 × 2.5 ml (2-11 y.) | 2 × 2.5 ml (2-11 y.) |
| | 1 × 5 ml (12-17 y.) | 2 × 5 ml (12-17 y.) |
| Eos < 5 per hpf (< 16/mm²) | 61.5 % | 88.5 % |
| Eos = 0 per hpf (0/mm²) | 53.8 % | 84.6 % |

It turned out that the high dose budesonide solution resulted in very high percentage of histological remission as well as histological deep remission after a treatment of 12 weeks only. The low dose resulted also in a remission substantially higher than treatment with placebo, but it seems that a high dosage budesonide treatment is better for obtaining a quick remission.

The study using the suspension described herein (BUU-5/EEA) is a similar study to a study assessing efficacy and safety of an oral budesonide suspension in a comparable paediatric patient population suffering EoE. (GUPTA et al., Clinical Gastroenterol. and Hepatol. 2015, vol. 13, No. 1, pp 66-67). Here, a dose of 1.4 and 2.0 mg was given in a 7 ml and 10 ml volume either in a once daily or in a twice daily dosing regimen. Similarly to the study BUU-5/EEA, efficacy was analysed by measuring the number of eosinophils per hpf. However, efficacy outcomes were defined and named slightly differently: Histological response was defined as eos ≤ 6 per hpf, histological remission as eos ≤ 1 per hpf (see Table 3).

**Table 3**

| | Gupta | |
|---|---|---|
| Treatment regimen | OD | BID |
| Daily dose | **1.4** - **2.0 mg** | **2.8** - **4.0 mg** |
| Volume | 1 × 7 ml (2-9 y.) | 2 × 7 ml (2-9 y.) |
| | 1 × 10 ml (10-18 y.) | 2 × 10 ml (10-18 y.) |
| Eos ≤ 6 per hpf | 52.6 % | 94.1 % |
| Eos ≤ 1 per hpf | 42.1 % | 76.5 % |

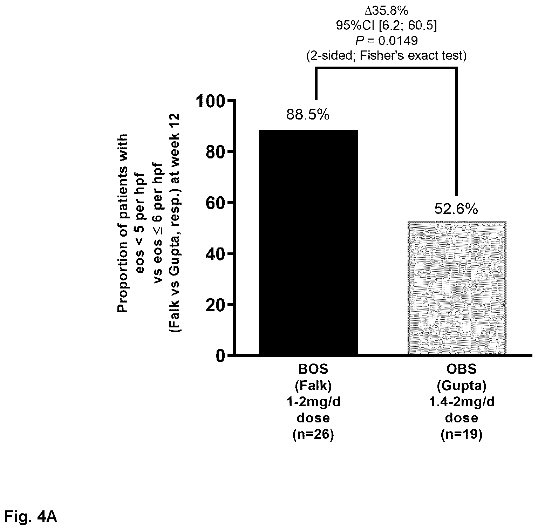

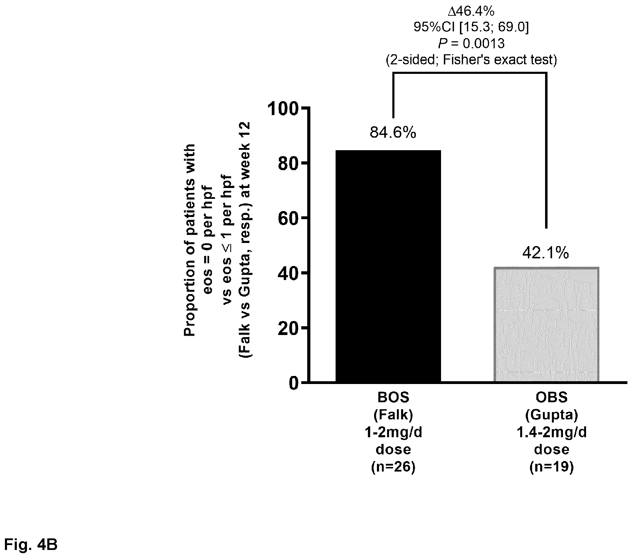

Surprisingly, with only around half the dose of budesonide similar and even better results could be obtained in both dosing regimens (OD and BID) with the budesonide oral suspension according to the invention in comparison to the Gupta study, even though the criteria were more stringent in the BUU-5/EEA study. Adjusted to the daily dose exposure of either 1.0 -2.0 mg according to the invention and 1.4 mg - 2.0 mg (as prior art) this reflects a doubling of efficacy in both histological response and remission. The results of the Gupta study are compared with the present invention (Falk) which are shown in Figures 4A and B. In Fig. 4A the proportion of patients with eos <5 per hpf vs eos ≤6 per hpf is shown and in Fig. 4B the proportion of patients with eos = 0 per hpf vs eos ≤1 per hpf is shown.

### Example 4 (stability of the orally applicable suspension)

One of the difficulties of a suspension is that such a suspension must be stable in various respects. On the one hand budesonide is stable against degradation when it is not in an aqueous environment. In solution or suspension, however, budesonide may be degraded which leads to undesired degradation products of budesonide which are not more highly pharmacologically active.

Another problem is that suspensions are under the risk of microbial degradation. The addition of preservants may, however, not be desired in particular when the suspension is designed for children or adolescents. The orally applicable suspension according to the invention has therefore been tested with regard to its stability over several months. The results are shown in Table 4. It is evident that the orally applicable suspension according to the present invention is surprisingly stable which allows a convenient handling in practice.

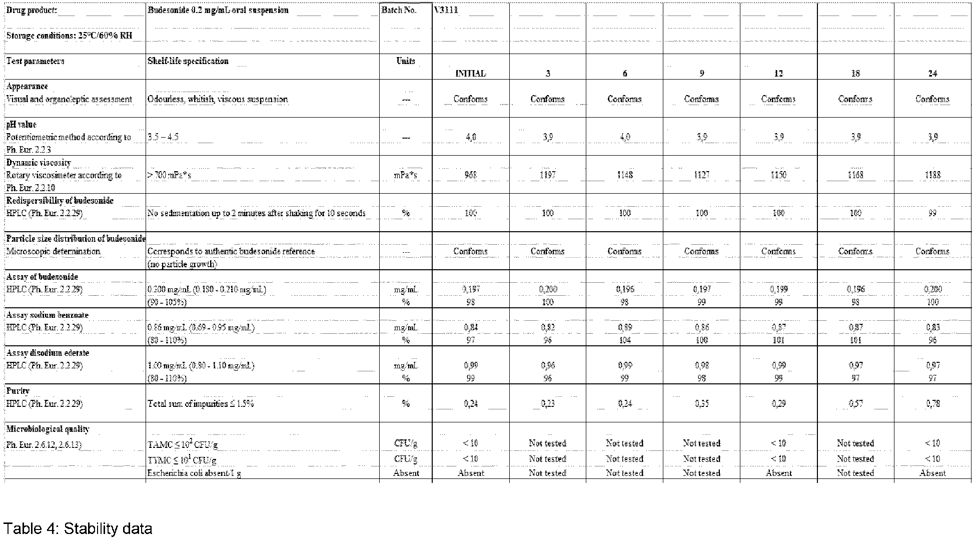

### Example 5 (adherence of active agent to the oesophagus)

In order to show superior adherence of the pharmacologically active agent to the esophagus an experiment with radioactively labeled microspheres was performed. The suspension according to the invention having 0.2 mg budesonide per milliliter was radioactively labeled with the gamma emitting nuclide ¹¹¹In. The orally applicable suspension according to the invention was compared with a Budesonide orodispersible tablet (ODT) designated Jorveza which was also labeled with Indium (¹¹¹In).

The passage of the labeled product (OS vs. ODT) was observed in a scintigraphy and the following conclusions can be drawn from the experiment:
- The transit time of the orally applicable suspension (OS) according to the invention is substantilly longer than the time range expected for non - viscous liquids which shows the surprising favorable properties of the OS for oesophageal targeting.
- When an orodispersible tablet cannot be given to the patient due to his/her age the orally applicable suspension according to the invention provides a good alternative to ODT as shown by the scintigraphy results.

## Claims

1. Orally applicable suspension comprising
a) topically acting micronized crystalline budesonide,
b) an aqueous base,
c) at least one viscosity-increasing agent selected from a methylcellulose or a derivative thereof,
d) a buffering agent which allows the adjustment of the final suspension to a pH value of 3.5 to 4.5,
e) an antimicrobial preservative,
f) a suspension stabilizing agent, in particular ethylenediaminetetramethyl acetic acid,
whereby the suspension has a dynamic viscosity in the range of 800 mPa*s to 2000 mPa*s.

2. Orally applicable suspension according to claim 1 wherein the final suspension contains a concentration of 0.1 mg/ml to 1.0 mg/ml budesonide.

3. Orally applicable suspension according to claim 2 wherein the budesonide concentration is 0.2 mg/ml.

4. Orally applicable suspension according to any of claims 1 to 3 wherein at least 95% of the budesonide microparticles have a diameter of less than 10 µm.

5. Orally applicable suspension according to any of claims 1 to 4 wherein the dynamic viscosity is in the range of 1000 to 1500 mPa*s, preferably in the range of 1000-1200 mPa*s.

6. Orally applicable suspension according to any of claims 1 to 5 which contains as sweetener sucrose.

7. Orally applicable suspension according to any of claims 1 to 6 which contains as flavoring agent a cassis aroma.

8. Orally applicable suspension according to any of claims 1 to 7 which contains no surfactant and no antioxidant.

9. Orally applicable suspension according to any of claims 1 to 8 which contains no other viscosity-increasing agent except methylcellulose or a derivative thereof.

10. Orally applicable suspension according to any of claims 1 to 9 for use in the treatment of eosinophilic esophagitis of a patient having an age of about 1 year to about 17 years whereby 0.5 to 1.0 mg budesonide is administered in 2.5 to 5.0 ml suspension once or twice a day.

11. Orally applicable suspension for use according to claim 10 for maintaining remission whereby the orally applicable suspension is applied for at least 48 weeks.

12. Method for the preparation of an orally applicable suspension according to claims 1 to 9 whereby
in a first process step the aqueous base is mixed with the viscosity-increasing agent whereby a suspension without pharmaceutically active agent is produced by temperature treatment, in a second step an aqueous solution containing a buffering agent, an antimicrobial preservative and a suspension stabilizing agent is prepared,
in a third step the suspension obtained from step one is mixed with the solution obtained in step two and
in a fourth step the micronized glucocorticosteroid is suspended under stirring into the mixture of step three and finally the flavoring agent is added before the orally applicable suspension is filled in a light protected bottle.

13. Orally applicable suspension according to any of claims 1 to 9 obtainable by a process according to claim 12.
